# EUROPÄISCHE PATENTSCHRIFT

(11) **EP 0 278 384 B1**
(45) Veröffentlichungstag und Bekanntmachung des Hinweises auf die Patenterteilung: **29.09.1993**
(21) Anmeldenummer: 88101560.6
(22) Anmeldetag: 04.02.1988
(51) Int. Cl.: C07C 31/133, C07C 33/16, C07C 43/115, C07C 43/162, C07C 29/136, C07C 67/343, C07C 41/16, C07C 29/17, A61K 7/46

(54) **Alkohole und Ether mit Cyclododecyl- und Cyclododecenylgruppen, deren Herstellung und Verwendung als Duftstoffe**
Alcohols and ethers with cyclododecyl and cyclododecenyl groups, their preparation and use as parfuming agents
Alcools et éthers contenant des groupements cyclododécyles et cyclododécényles leurs préparation et utilisation comme agents parfumants

(30) Priorität: 06.02.1987 DE 3703585
(43) Veröffentlichungstag der Anmeldung: 17.08.1988
(73) Patentinhaber: Consortium für elektrochemische Industrie GmbH, D-81379 München (DE)
(72) Erfinder: Hafner, Walter, Dr., D-8196 Eurasburg (DE); Gebauer, Helmut, Dr., D-8000 München 71 (DE); Markl, Erich, D-8000 München 21 (DE); Regiert, Marlies, D-8000 München 22 (DE)

(56) Entgegenhaltungen:
- EP-A- 0 213 633
- DE-A- 2 630 835
- DE-A- 2 852 344

## Beschreibung

Es sind zahlreiche Duftstoffe mit Cyclododecyl- und Cyclododecenylgruppen bekannt. Es handelt sich dabei vorwiegend um Ether und Ketone, aber auch Alkohol-, Ester- oder Nitrilderivate sind darunter zu finden. Diese Verbindungen gehören allgemein zur Gruppe der Holzriechstoffe, wobei jedoch jede Verbindung ihre eigene Note besitzt.

Aufgabe der Erfindung ist das Auffinden neuer Holzriechstoffe mit eigenem Charakter, die gute Haftfähigkeit, einen geringen Schwellenwert und eine niedrige Verdünnungsgrenze zeigen.

Gegenstand der Erfindung sind Verbindungen der allgemeinen Formeln 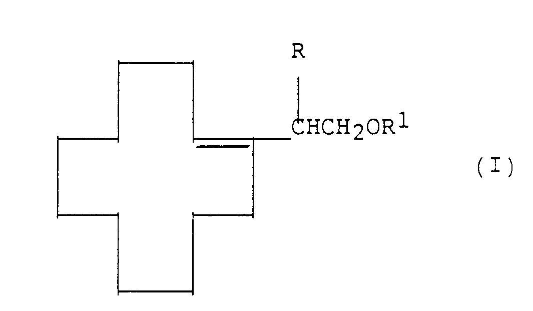 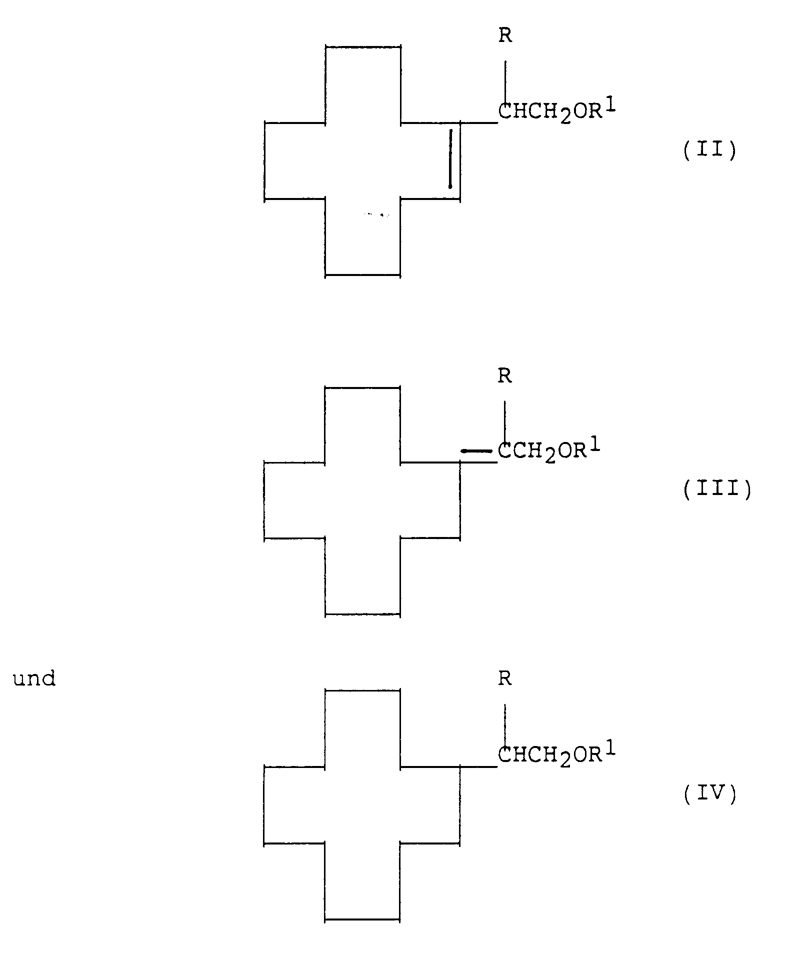
wobei R ein Methyl- oder Ethylrest und R¹ ein Wasserstoff-, Methyl- oder Ethylrest sein kann.

Ein Verfahren zur Herstellung von Verbindungen der allgemeinen Formeln 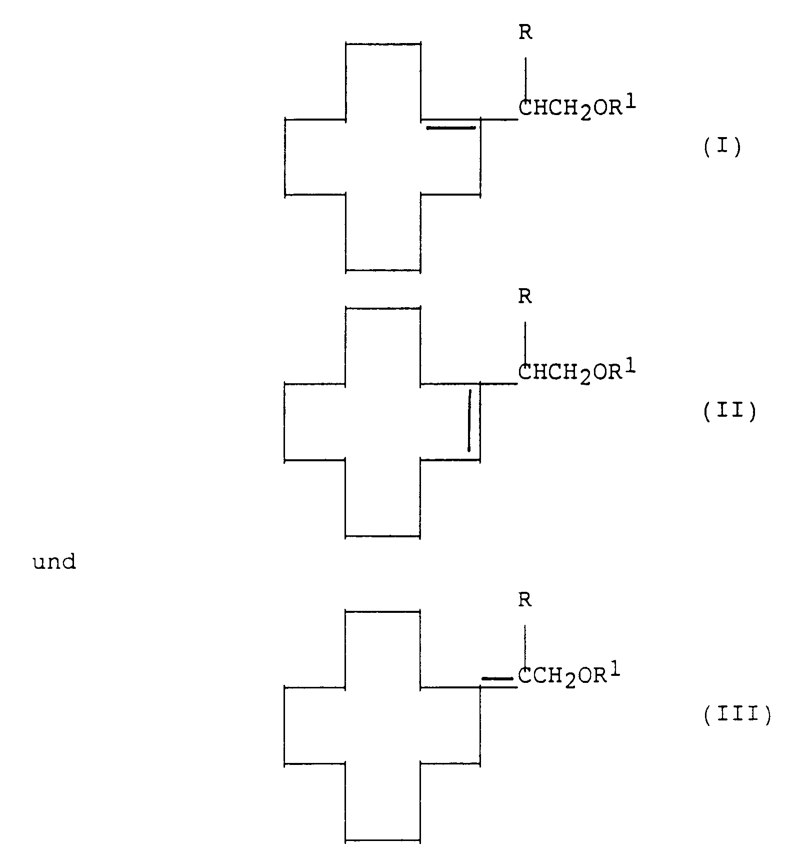
wobei R ein Methyl- oder Ethylrest und R¹ ein Wasserstoff-, Methyl- oder Ethylrest sein kann, ist dadurch gekennzeichnet, daß
a) Cyclododecanon mit Br-CHR-COOEt (R hat die oben dafür gegebene Bedeutung) in Gegenwart von Zink umgesetzt wird und nach erfolgter Wasserabspaltung
b) das Reaktionsprodukt gemäß a) mit Lithiumaluminiumhydrid reduziert wird und gegebenenfalls in an sich bekannter Weise verethert wird.

Cyclododecanon und Br-CHR-COOEt ( R hat die oben dafür gegebene Bedeutung) sind handelsübliche Verbindungen oder durch einfache Verfahren leicht zugänglich. Die Umsetzung erfolgt vorzugsweise bei Temperaturen im Bereich von 80°C bis 110°C in für Reformatski-Reaktionen gebräuchlichen inerten organischen Lösungsmitteln wie Aromaten beispielsweise Toluol und Ethern beispielsweise Diethylether. Kräftiges Rühren, gegebenenfalls auch die Anwendung von Ultraschall sind für die Reaktion förderlich. Nach Zersetzung mit Mineralsäuren wie Salzsäure erfolgt Wasserabspaltung mit Säuren wie Phosphorsäure oder Toluolsulfonsäure, gegebenenfalls unter Zuhilfenahme eines Veresterungsreagenzes wie Essigsäureanhydrid. Das Reaktionsprodukt gemäß a) wird mit Lithiumaluminiumhydrid reduziert. Es werden für den jeweiligen Rest R Isomerengemische bestehend aus den jeweiligen Verbindungen der allgemeinen Formeln I, II und/oder III erhalten, die beispielsweise durch fraktionierende Destillation, Ausfrieren oder chromatographische Methoden getrennt werden können. Die Zusammensetzung der Isomerengemische richtet sich weitgehend nach den gewählten Reaktions- und Aufarbeitungsbedingungen bei der Synthese.

Ein Verfahren zur Herstellung von Verbindungen der allgemeinen Formel 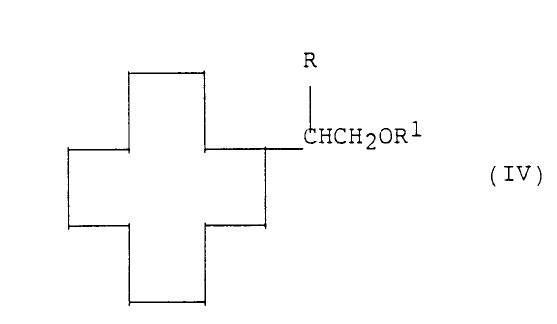
wobei R ein Methyl- oder Ethylrest und R¹ ein Wasserstoff-, Methyl- oder Ethylrest sein kann, ist dadurch gekennzeichnet, daß das Reaktionsprodukt gemäß a) oder b) mit Reduktionskatalysator und Wasserstoff umgesetzt wird und dieses Reaktionsprodukt gegebenenfalls in an sich bekannter Weise verethert wird.

Als Reduktionskatalysatoren werden bevorzugt Palladium auf Aktivkohle oder Raney-Nickel verwendet.

Die erfindungsgemäßen Alkohole lassen sich nach bekannten Verfahren, beispielsweise die Williamsonsche Ethersynthese, in die Methyl- oder Ethylether umwandeln, die ebenfalls Holzriechstoffe sind.

Bezüglich weiterer Einzelheiten wird ausdrücklich auf die Beispiele verwiesen.

### Beispiel 1

### 2-Cyclododecenyl-propan-1-ole

In einem 2l Dreihalskolben mit Magnetrührer, Thermometer, Rückflußkühler und Tropftrichter mit Inertgasanschluß wurden 150ml Toluol und 128g granulares Zink vorgelegt und unter Argon auf 95°C erwärmt. Im Verlauf einer Stunde wurde eine Mischung von 334g Cyclododecanon, 330g Brompropionsäureethylester, 350ml Toluol und 50ml Ether zugetropft. Die Temperatur wurde 4 Stunden auf 95-100°C gehalten. Nach Abkühlung wurde der Ansatz auf Eis gegossen, mit konz. Salzsäure angesäuert und die Schichten getrennt. Die wässerige Phase wurde einmal mit 200ml tert.-Butylmethylether ausgeschüttelt und dann verworfen. Von den vereinigten organischen Schichten wurden die Lösungsmittel weitgehend abdestilliert. Der Rückstand wurde mit 300ml Xylol, 210g Essigsäureanhydrid und 5g p-Toluolsulfonsäure versetzt und 3 Stunden unter Rückfluß gekocht. Dann wurde vorsichtig 5g Soda zugesetzt, nach Abklingen der Gasentwicklung Essigsäure, Essigsäureanhydrid und Lösungsmittel im Teilvakuum abdestilliert und der Rückstand filtriert. Anschließend wurde die Destillation im Vakuum fortgesetzt. Nach einem Vorlauf gingen von 95-115°C/0,1-0,3mbar 390g Isomerengemisch von 2-cyclododecenylpropionsäureethylestern über.
200g dieses Isomerengemisches wurden unter Argon bei 30-40°C zu einer Lösung bzw. Suspension von 20g Lithiumaluminiumhydrid in 400ml Tetrahydrofuran getropft. Die Mischung wurde 2 Stunden bei dieser Temperatur und weitere 4 Stunden bei 65°C gehalten. Nach Kühlung wurde der Ansatz auf Eis gegossen, mit Salzsäure angesäuert und die obere Schicht abgetrennt. Die wässerige Phase wurde einmal mit tert.-Butylmethylether ausgeschüttelt und verworfen. Die vereinigten organischen Schichten wurden mit verdünnter Sodalösung ausgeschüttelt, über festem K₂CO₃ getrocknet und über eine Vigreux-Kolonne destilliert. Es wurden 160g eines Isomerengemisches (Siedepunkt 103°C bei 0,07mbar) der Zusammensetzung 90 Gew.-% Verbindung der allgemeinen Formel I, 9 Gew.-% Verbindung der allgemeinen Formel II und weniger als 1 Gew.-% Verbindung der allgemeinen Formel III, wobei R=CH₃ und R¹=H, erhalten.

Geruchsnote: Stark ambrierter Holzkörper mit blumigen Aspekten von Muguet.

### Beispiel 2

### 2-Cyclododecenylpropylmethylether

15g der gemäß Beispiel 1 hergestellten Verbindung wurden unter Argonspülung zu einer Suspension von 4,4g Natriumhydrid in 150ml trockenem Tetrahydrofuran gegeben und 2 Stunden unter Rückfluß erhitzt. Dann wurden 8,5g Dimethylsulfat in 50ml Tetrahydrofuran zugegeben und eine weitere Stunde unter Rückfluß erhitzt. Dann wurde der Ansatz auf Eis gegossen, das organische Material mit tert.-Butylmethylether aufgenommen und mit 10%-iger NaOH ausgeschüttelt. Nach Trocknung mit K₂CO₃ wurden die Lösungsmittel entfernt und das Produkt über eine kleine Vigreux-Kolonne destilliert.

Es wurden 10g Cyclododecenylpropylmethylether (Siedepunkt 90-97°C/0,2mbar) erhalten.
Geruchsnote: holzig, blumig, ambriert, erdig,an Vetiveröl erinnernd.

### Beispiel 3

### 2-Cyclododecylpropan-1-ol

45g des Isomerengemisches von 2-Cyclododecenylpropan-1-olen gemäß Beispiel 1 wurden mit 2g Raney-Nickel und 10ml Ethanol im Schüttelautoklaven 7 Stunden mit 200bar Wasserstoff auf 200°C erhitzt. Das Reaktionsgemisch wurde nach Entfernung von Katalysator und Lösungsmittel destilliert. Bei 105-110°C und 0,7mbar erhielt man 35g 2-Cyclododecylpropan-1-ol.

Geruchsnote: Etwas blumiger, warmer, schwerer Holzduft.

### Beispiel 4

### 2-Cyclododecylpropylethylether

15g 2-Cyclododecylpropan-1-ol wurden mit 4g Natriumhydrid in 150ml Tetrahydrofuran unter Luftausschluß zwei Stunden bei Siedetemperatur gerührt. Dann wurden 6g Diethylsulfat zugetropft und weitere zwei Stunden unter Rückfluß erhitzt. Die abgekühlte Reaktionsmischung wurde auf Eis gegossen und mit 20ml 10%-iger NaOH, sowie 50ml tert.-Butylmethylether ausgerührt. Die wäßrige Schicht wurde nochmals mit tert.-Butylmethylether, die vereinigten organischen Schichten nochmal mit 10%-iger NaOH ausgeschüttelt und destilliert. Nach Entfernung der Lösungsmittel wurden von 92-95°C bei 0,2mbar 9,5g 2-Cyclododecylpropylethylether erhalten.
Geruchsnote: ambriert holzig, herb-krautig, an Muskateller-Salbeiöl erinnernd.

### Beispiel 5

### 2-Cyclododecenylbutan-1-ole

Unter Luftausschluß wurden 26g Zink-pulver und 40ml trockenes Toluol auf 90°C erhitzt. Im Verlauf einer Stunde wurde unter kräftigem Rühren eine Mischung von 78g 2-Brombuttersäureethylester, 73g Cyclododecanon, 200ml trockenes Toluol und 20ml trockener Diethylether zugetropft. Anschließend wurde der Ansatz weitere 5 Stunden bei 81°C gerührt, dann gekühlt und auf eine Mischung aus Eis und Salzsäure gegossen. Die organische Schicht wurde abgetrennt, die wäßrige wurde mit tert.-Butylmethylether ausgeschüttelt und verworfen. Die vereinigten organischen Schichten wurden nochmals mit verdünnter Salzsäure und Wasser ausgeschüttelt, bevor die Lösungsmittel abdestilliert wurden. Der Rückstand wurde mit 100ml Mesitylen, 100ml Essigsäureanhydrid und 2g Phosphorsäure erhitzt, wobei langsam Essigsäure abdestillierte. Anschließend wurde der ganze Ansatz destilliert. Es wurden bis 90°C/0,1mbar 16g Cyclododecanon und bei 95-105°C/0,1mbar 42g eines Isomerengemisches von 2-Cyclododecenylbuttersäureethylester erhalten. Diese 42g Isomerengemisch wurden unter Luftausschluß in 150ml trockenem Tetrahydrofuran mit 3g Lithiumaluminiumhydrid 7 Stunden bei 45-50°C gerührt. Dann wurde mit Eis und Salzsäure zersetzt, das Produkt mit Ether aufgenommen, neutral gewaschen und destilliert. Nach einem kleinen Vorlauf von Cyclododecanol wurden von 115-118°C/0,2mbar 21g 2-Cyclododecenylbutan-1-ole erhalten.
Geruchsnote: Geruch von Tabak, Cedernblätteröl, Holz und Ambra.

## Patentansprüche

1. Verbindungen der allgemeinen Formeln 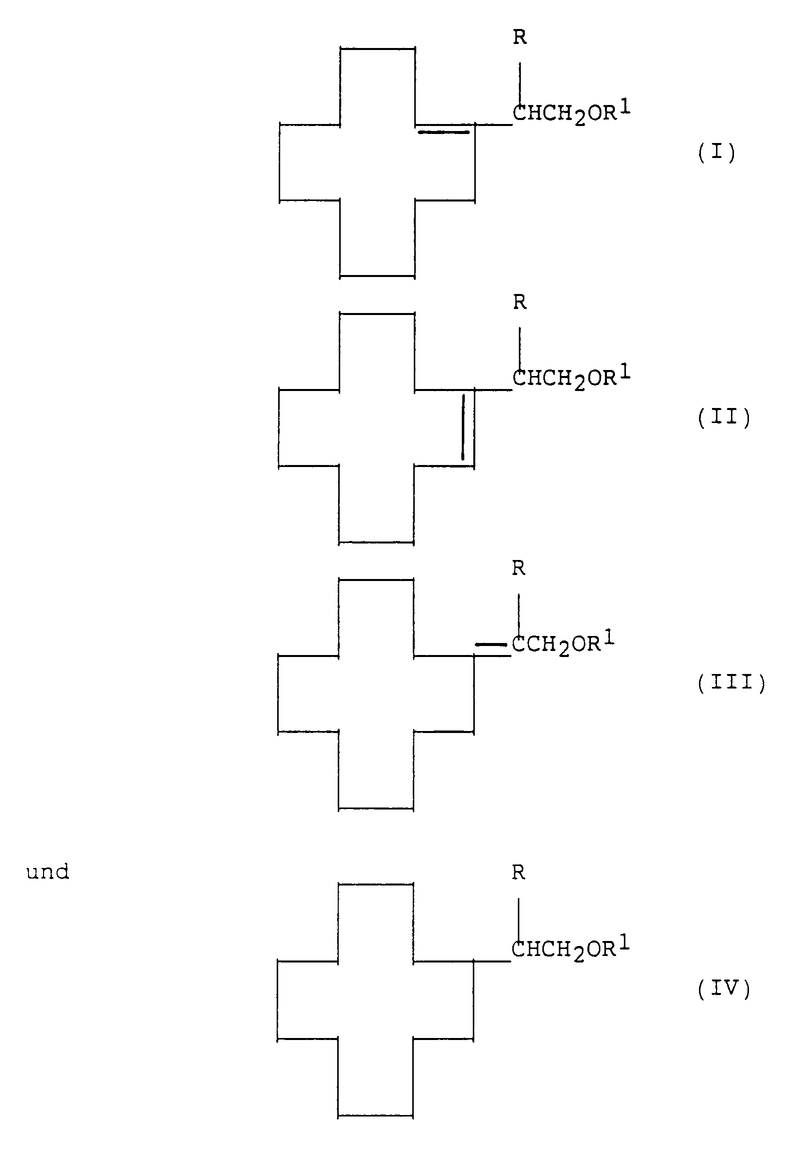 wobei R ein Methyl- oder Ethylrest und R¹ ein Wasserstoff-, Methyl- oder Ethylrest sein kann.

2. Verfahren zur Herstellung von Verbindungen der allgemeinen Formeln 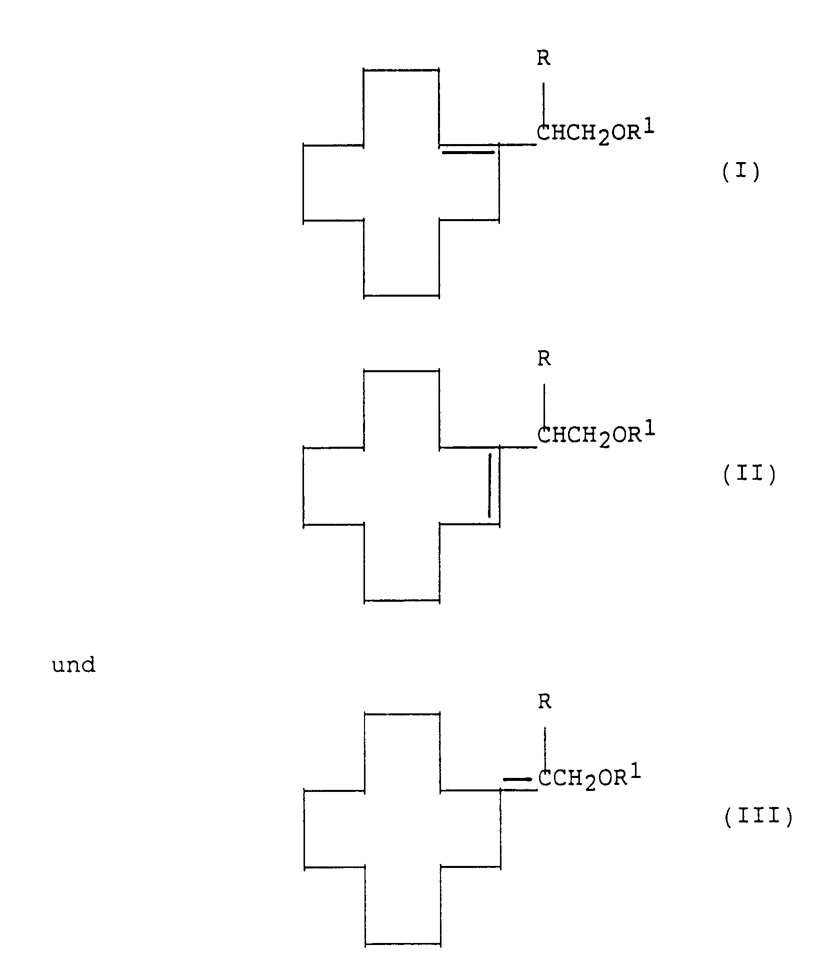 wobei R ein Methyl- oder Ethylrest und R¹ ein Wasserstoff-, Methyl- oder Ethylrest sein kann, dadurch gekennzeichnet, daß
a) Cyclododecanon mit Br-CHR-COOEt (R hat die oben dafür angegebene Bedeutung) in Gegenwart von Zink umgesetzt wird und nach erfolgter Wasserabspaltung
b) das Reaktionsprodukt gemäß a) mit Lithiumaluminiumhydrid reduziert wird und gegebenenfalls in an sich bekannter Weise verethert wird.

3. Verfahren zur Herstellung von Verbindungen der allgemeinen Formel 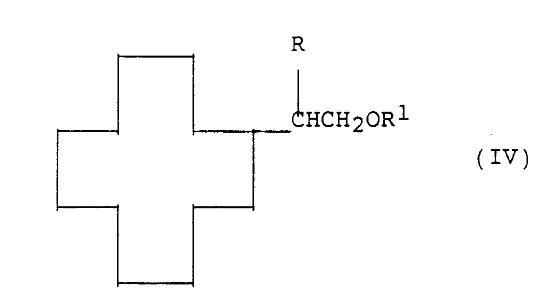 wobei R ein Methyl- oder Ethylrest und R¹ ein Wasserstoff-, Methyl- oder Ethylrest sein kann, dadurch gekennzeichnet, daß das Reaktionsprodukt gemäß a) oder b) mit Reduktionskatalysator und Wasserstoff umgesetzt wird und dieses Reaktionsprodukt gegebenenfalls in an sich bekannter Weise verethert wird.

4. Verwendung der Verbindung nach Anspruch 1 oder von Gemischen von Verbindungen nach Anspruch 1 als Duftstoffe.

## Claims

1. Compound of the formula 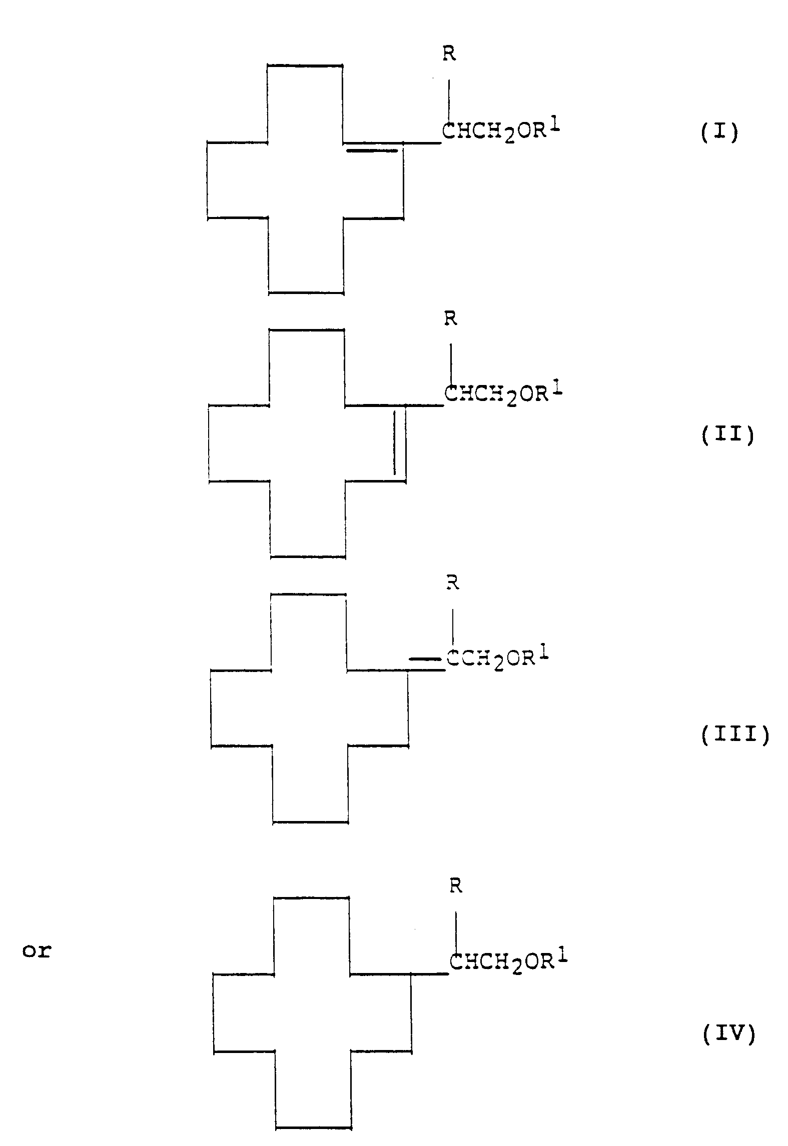 where R may be a methyl or ethyl radical and R¹ may be a hydrogen, methyl or ethyl radical

2. Process for the preparation of a compound of the formula 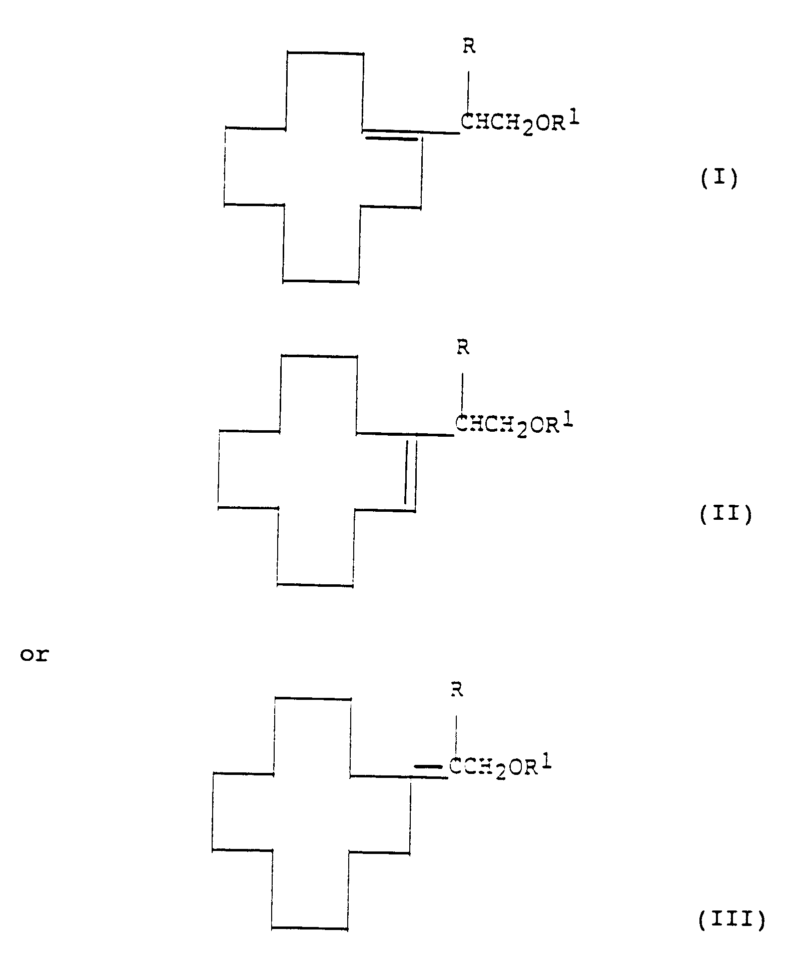 where R may be a methyl or ethyl radical and R¹ may be a hydrogen, methyl or ethyl radical, characterised in that
a) cyclododecanone is reacted with Br-CHR-COOEt (R has the abovementioned meaning for R) in the presence of zinc, and, when the elimination of water is complete,
b) the reaction product from a) is reduced using lithium aluminium hydride, and, if appropriate, the product is etherified in a fashion which is known per se.

3. Process for the preparation of a compound of the formula 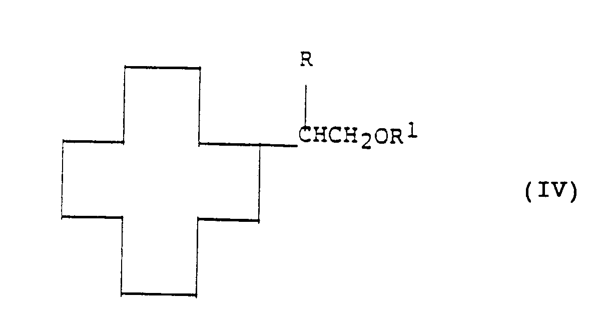 where R may be a methyl or ethyl radical and R¹ may be a hydrogen, methyl or ethyl radical, characterised in that the reaction product from a) or b) is reacted with a reduction catalyst and hydrogen, and, if appropriate, this reaction product is etherified in a fashion which is known per se.

4. Use of the compound according to Claim 1 or mixtures of compounds according to Claim 1 as scents.

## Revendications

1. Composés de formules générales suivantes : 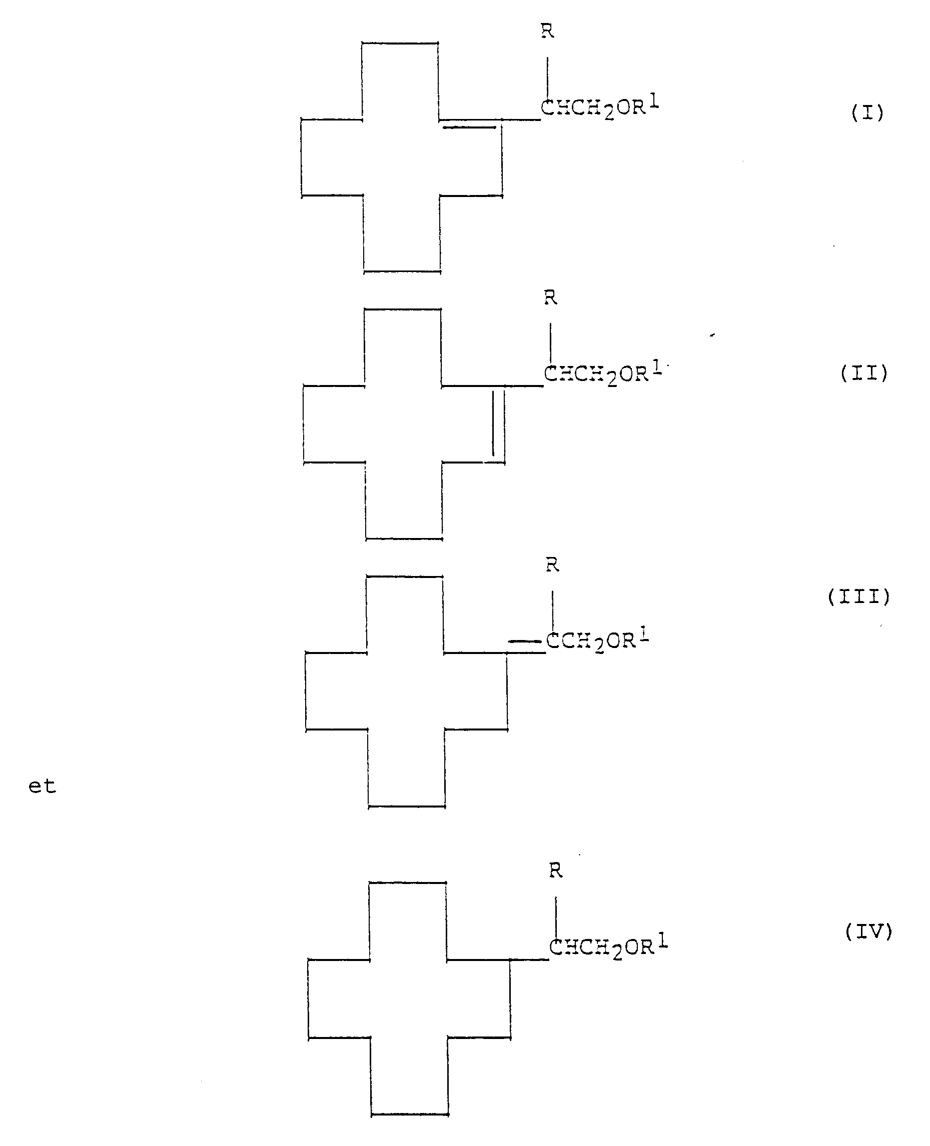 formules dans lesquelles R peut être le radical méthyle ou éthyle et R¹ un atome d'hydrogène ou le radical méthyle ou éthyle.

2. Procédé de préparation de composés de formules générales : 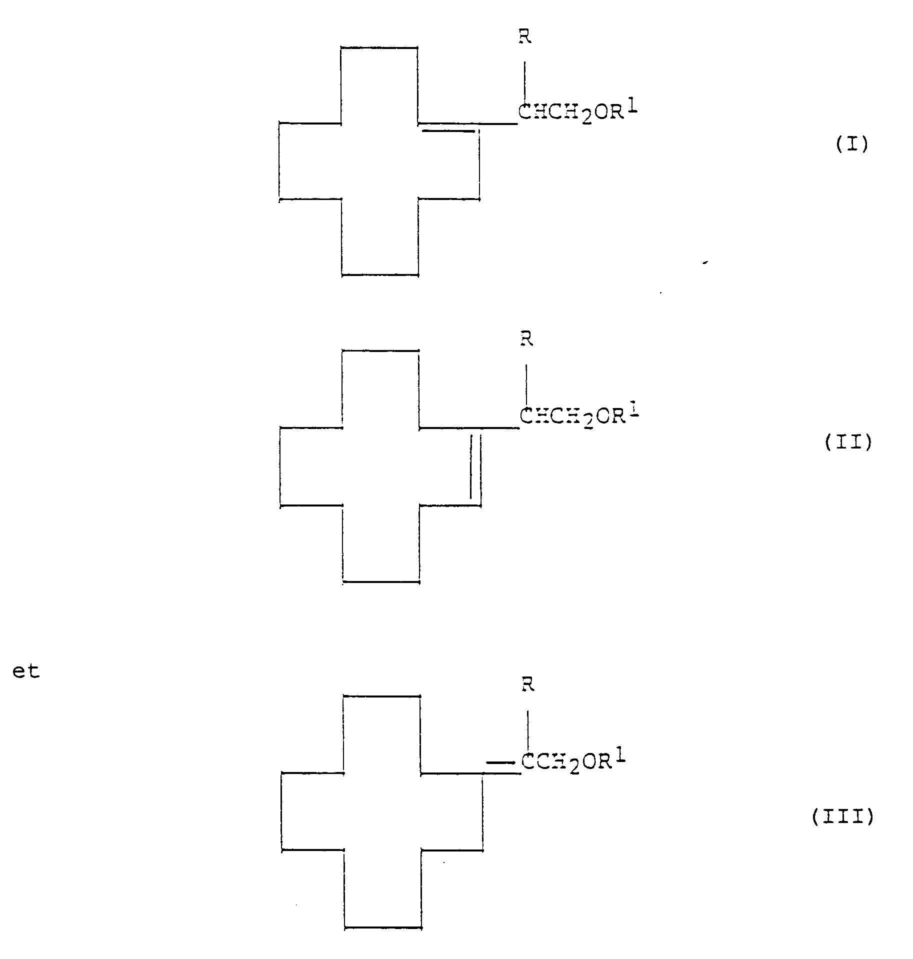 R pouvant représenter le radical méthyle ou éthyle et R¹ un atome d'hydrogène ou le radical méthyle ou éthyle, procédé caractérisé en ce que :
a) on fait réagir de la cyclododécanone avec un composé Br-CHR-COOEt (R ayant la signification ci-dessus) en présence de zinc, et après élimination de l'eau,
b) on réduit le produit ainsi formé avec de l'hydrure de lithium et aluminium et le cas échéant on éthérifie de manière connue le produit obtenu.

3. Procédé de préparation de composés de formule générale : 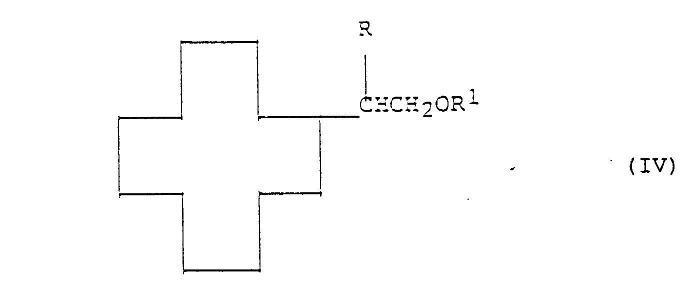 R pouvant désigner le radical méthyle ou éthyle et R¹ un atome d'hydrogène ou le radical méthyle ou éthyle, procédé caractérisé en ce que l'on fait réagir avec de l'hydrogène en présence d'un catalyseur de réduction le produit formé en a) ou b) selon la revendication 2, et le cas échéant on éthérifie de manière connue le produit obtenu.

4. L'emploi comme parfums des composés de la revendication 1 ou de mélanges de plusieurs de ces composés.
